# EUROPEAN PATENT SPECIFICATION

(11) **EP 1 824 419 B1**
(45) Date of publication and mention of the grant of the patent: **20.04.2016**
(21) Application number: 05853997.4
(22) Date of filing: 14.12.2005
(51) Int. Cl.: A61F 2/46, A61F 2/30, A61F 2/36, A61F 2/38, A61F 2/28

(54) **MODULAR IMPLANT SYSTEM**
MODULARES IMPLANTATSYSTEM
SYSTEME D'IMPLANT MODULAIRE

(30) Priority: 17.12.2004 US 637015 P; 31.10.2005 US 732402 P; 31.10.2005 US 731999 P
(43) Date of publication of application: 29.08.2007
(73) Proprietor: DePuy Products, Inc., Warsaw, IN 46581 (US)
(72) Inventor: HECK, Robert, K., Memphis, TN 38117 (US); HAZEBROUCK, Steven, A., Winona Lake, IN 46590 (US)
(74) Representative: Belcher, Simon James
(86) International application number: PCT/US2005/045197
(87) International publication number: WO 2006/065879

(56) References cited:
- EP-A2- 1 358 860
- WO-A1-02/05732
- US-A- 4 384 373
- US-A- 4 502 160
- US-A- 4 676 797
- US-A- 4 787 907
- US-A- 4 787 907
- US-A- 4 938 768
- US-A- 5 358 524
- US-A- 5 658 349
- US-B1- 6 322 591
- US-B1- 6 613 092
- US-B1- 6 712 858

## Description

### CROSS-REFERENCE TO RELATED APPLICATION

The present invention claims priority to United States Provisional Patent Application Serial No. 60/637,015, filed on December 17, 2004 by Robert K. Heck and Stephen A. Hazebrouck and entitled "Modular Implant System and Method with Diaphyseal Implant," United States Provisional Patent Application Serial No. 60/732,402, filed on October 31, 2005 by Robert K. Heck and Stephen A. Hazebrouck and entitled "Modular Implant System and Method with Diaphyseal Implant and Adapter," and United States Provisional Patent Application Serial No. 60/731,999, filed on October 31, 2005 by Robert K. Heck and Stephen A. Hazebrouck, and entitled "Modular Diaphyseal and Collar Implant".

### BACKGROUND OF THE INVENTION

The present invention relates generally to prosthetic joints and, more particularly, to modular orthopaedic lower extremity implant systems.

The knee joint basically consists of the bone interface of the distal end of the femur and the proximal end of the tibia. Appearing to cover or at least partially protect this interface is the patella which is a sesamoid bone within the tendon of the long muscle (quadriceps) on the front of the thigh. This tendon inserts into the tibial tuberosity and the posterior surface of the patella is smooth and glides over the femur.

The distal femur is configured with two knob like processes (the medial condyle and the lateral condyle) which are substantially smooth and which articulate with the medial plateau and the lateral plateau of the tibia, respectively. The plateaus of the tibia are substantially smooth and slightly cupped thereby providing a slight receptacle for receipt of the femoral condyles.

The hip joint consists of the bone interface of the proximal end of the femur and the acetabulum of the hipbone. The proximal femur is configured with a ball-shaped head, which is received within and articulates against the cup-shaped cavity defined by the acetabulum.

When the knee or hip joint is damaged whether as a result of an accident or illness, a prosthetic replacement of the damaged joint may be necessary to relieve pain and to restore normal use to the joint. Typically the entire joint is replaced by means of a surgical procedure, which involves removal of the surfaces of the corresponding damaged bones and replacement of these surfaces with prosthetic implants. This replacement of a native joint with a prosthetic joint is referred to as a primary total-knee arthroplasty and primary total-hip arthroplasty.

On occasion, the primary prosthesis fails. Failure can result from many causes, including wear, aseptic loosening, osteolysis, ligamentous instability, arthrofibrosis and patellofemoral complications. When the failure is debilitating, revision surgery may be necessary. In a revision, the primary prosthesis is removed and replaced with components of a revision prosthetic system.

Implant systems for both primary and revision applications are available from a variety of manufacturers, including DePuy Orthopaedics, Inc. of Warsaw, Indiana. DePuy and others offer several different systems for both primary and revision applications. For example, DePuy Orthopaedics offers the P.F.C. SIGMA® Knee System, the LCS® Total Knee System, and the S-ROM Modular Total Knee System. Each of these orthopaedic knee systems includes several components, some appropriate for use in primary knee arthroplasty and some appropriate for use in revision surgery.

DePuy Orthopaedics also offers other orthopaedic implant systems for other applications. One such system is the LPS System. The LPS System is provided for use in cases of neoplastic diseases (e.g., osteosarcomas, chondrosarcomas, giant cell tumors, bone tumors) requiring extensive resections and replacements of the proximal and/or distal femur, severe trauma, disease (e.g., avascular necrosis, osteoarthritis and inflammatory joint disease requiring extensive resection and replacement of the proximal and/or distal femur), and resection cases requiring extensive resection and replacement of the proximal, distal or total femur or proximal tibia (e.g., end-stage revision). Any of these conditions or a combination thereof can lead to significant amounts of bone loss. The LPS System provides components that can replace all or significant portions of a particular bone, such as the femur or tibia. The DePuy LPS System is described more fully in United States Patent Application Serial No. 10/135,791, entitled "Modular Limb Preservation System", filed April 30, 2002 by Hazebrouck et al., U.S. Pat. Publication No. US2003/0204267A1 (published October 30, 2003). Other companies also offer systems for similar indications.

The LPS system provides a comprehensive set of modular implants capable of addressing a wide range of orthopaedic conditions. Components of the LPS system can be combined in a variety of ways to account for variations in patient anatomy and differences in the amount of native bone remaining. As disclosed in U.S. Patent Publication No. US2003/0204267A1, the modular components can be combined to replace the proximal or distal femur, total femur, proximal tibia or the mid-shaft of a long bone. Similar systems can be used with other long bones, such as the bones of the upper arm.

Many of the combinations of components possible with the LPS system include stem components that are configured for implantation within the intramedullary canal of the remaining bone. Metaphyseal sleeves are available for use in the LPS system, as disclosed, for example, in U.S. Pat. App. Ser. No. 10/817,051, entitled " Modular Implant System with Fully Porous Coated Sleeve", filed on April 2, 2004 by Goodfried, Hazebrouck, Lester and Brown (U.S. Pat. Publication No. US2005/017883A1). However, in some instances, the stem components must be used with implant components that have replaced the entire articulating portion of the bone and the metaphysis of the bone. In some indications, the remaining native bone comprises the diaphysis or shaft of the long bone, and a metaphyseal sleeve cannot be used. The features of the preamble of cliam 1 are known from WO 02/05732 A1.

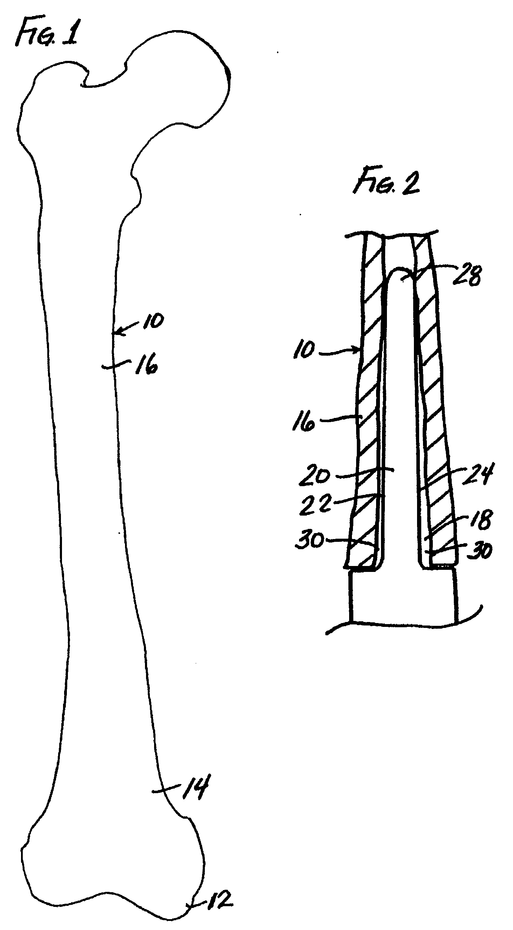

An example of a long bone is illustrated in FIG. 1; in FIG. 1, the bone 10 is the femur. FIG. 2 illustrates the femur of FIG. 1 after the distal articulating end 12 and metaphysis 14 of the bone 10 have been removed due to neoplastic disease, trauma, disease or as part of an end-stage revision. The diaphysis of the bone is illustrated at 16 in FIGS. 1-2.

As shown in FIG. 2, the intramedullary canal 18 of the diaphysis 16 of the long bone 10 generally tapers, while the implant stem extensions 20 generally have parallel sides, such as those shown at 22, 24. As a result, the implant stem extension 20 frequently contacts the native bone tissue at the free end or tip 28 of the stem extension 20, while leaving gaps 30 along much of the length of the stem extension 20. Although these gaps 30 could be filled with bone cement, for optimal fixation it is desirable to use porous coated stem extensions. Such porous coated stem extensions tend to bind before becoming fully seated. Consequently, in cases where the stem extension is porous coated to encourage bone ingrowth, the bone ingrowth is frequently limited to the free end 28 of the stem. With bone ingrowth limited to the free end of the stem extension, there is stress shielding of the bone surrounding the remainder of the stem extension, and a long lever arm is created; both of these effects can lead to early loosening of the implant. Additionally, when significant ingrowth does occur and the stem extension must subsequently be removed, the procedure can be difficult.

### SUMMARY OF THE INVENTION

The present invention addresses the need for an implant system that can be effectively used in the diaphyseal region of a long bone and discloses a surgical method for implanting a system in the diaphyseal region of a long bone.

The present invention addresses this need by providing a diaphyseal implant component according to the features of claim 1.

In addition, the present description reveals a method of replacing a portion of a long bone having an articulating surface, an intramedullary canal and a diaphysis spaced from the articulating surface. The method comprises resecting the bone to remove a portion of the bone and leaving at least a portion of the diaphysis of the bone. A tapered bore is prepared in the diaphysis of the bone. An implant is provided comprising a diaphyseal portion and a stem portion. The stem portion is inserted into the intramedullary canal and the diaphyseal portion is inserted into the tapered bore in the diaphysis of the bone.

In another aspect, the present invention addresses this need by providing an orthopaedic implant kit for replacing a portion of a long bone, the long bone having an articulation portion, a diaphysis and an intramedullary canal. The kit includes a plurality of modular stems shaped to be received in the intramedullary canal of the long bone. Each stem has a free end and an opposite end to be connected to another implant component. The kit also includes a plurality of modular diaphyseal components capable of being connected to the modular stems. Each diaphyseal component includes a first end, a second end for connection to a selected modular stem, and a porous tapered outer surface. The porous tapered outer surface has a minimum outer diameter at the second end and a maximum outer diameter spaced from the second end. The second tapered portion has the same outer dimension along two perpendicular transverse axes at its maximum outer diameter.

### BRIEF DESCRIPTION OF THE DRAWINGS

FIG. 1 is an anterior view of a left femur;
FIG. 2 is a cross-section of a portion of the diaphysis of the femur of FIG. 1, shown with a stem extension received in the intramedullary canal of the femur;
FIG. 3 is an elevation of a set of diaphyseal implant components of one embodiment of a set of orthopaedic implant components embodying the principles of the present invention;
FIG. 4 is an elevation of the set of diaphyseal implant components of FIG. 3, shown with the diaphyseal implant components turned ninety degrees about their longitudinal axes;
FIG. 5 is a longitudinal cross-section of one of the diaphyseal implant components of FIGS. 3-4 taken along line 5-5 of FIG. 3;
FIG. 6 is a longitudinal cross-section of another of the diaphyseal implant components of FIGS. 3-4, taken along line 6-6 of FIG. 3;
FIG. 7 is an end view of one of the diaphyseal implant components of FIGS. 3-4, taken along line 7-7 of FIG. 4;
FIG. 8 is an end view of one of the diaphyseal implant components of FIGS. 3-4, taken along line 8-8 of FIG. 3;
FIG. 9 is an exploded perspective view of a distal femoral implant assembly illustrating use of one of the diaphyseal implant components of FIGS. 3-4 in use with one style of stem extension;
FIG. 10 is an exploded perspective view similar to FIG. 9, but illustrating use of one of the diaphyseal implant components of FIGS. 3-4 in use with an adapter and a different style of stem extension;
FIG. 11 is a side view of a distal femoral implant assembly including one of the diaphyseal implant components of FIGS. 3-4 in use with a different style of stem extension;
FIG. 12 is a posterior view of the distal femoral implant assembly of FIG. 11;
FIG. 13 is a side view of a proximal femoral implant assembly including one of the diaphyseal implant components of FIGS. 3-4;
FIG. 14 is a perspective view of a proximal tibial implant assembly including one of the diaphyseal implant components of FIGS. 3-4;
FIG. 15 is a side view of an intercalary implant assembly including two of the diaphyseal implant components of FIGS. 3-4;
FIG. 15A is a side view of an intercalary implant assembly including one of the diaphyseal implant components of FIGS. 3-4;
FIG. 16 is a diagrammatic cross-section of a portion of the remaining portion of the diaphysis after a portion of the femur or long bone has been resected;
FIG. 17 illustrates the remaining resected diaphysis of FIG. 16 after a tapered bore has been prepared at the resection site of the bone; and
FIG. 18 illustrates the remaining resected diaphysis of FIG. 17 with an implant assembly including a diaphyseal implant component fully seated in the bone.

### DETAILED DESCRIPTION

A modular orthopaedic knee implant system incorporating the principles of the present invention is illustrated in the accompanying drawings. The illustrated modular orthopaedic knee implant system includes components of several existing orthopaedic knee implant systems, along with new components that provide the orthopaedic surgeon with greater flexibility in selecting the appropriate components to suit the needs of an individual patient. These patient needs can include factors such as individual anatomy and the condition of the native bone tissue.

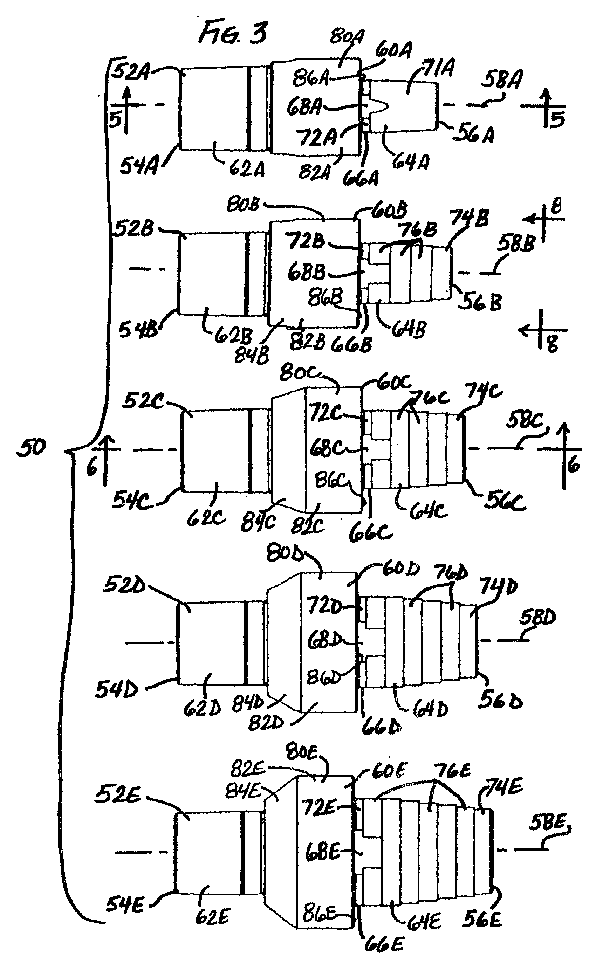

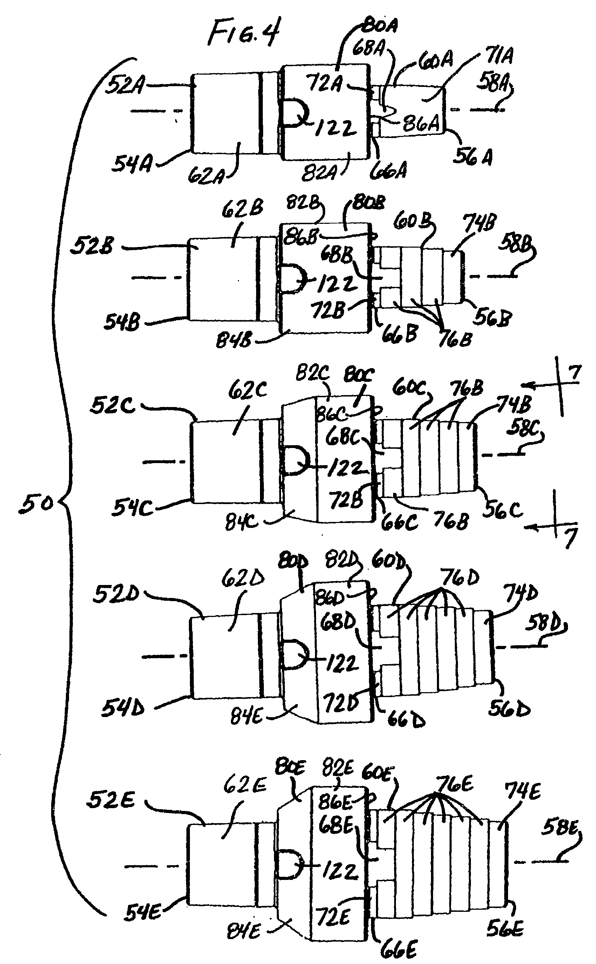

FIGS. 3-4 illustrate a set 50 of diaphyseal implant components that can be used in the system or kit of the present invention. The illustrated set 50 of diaphyseal implant components includes five sizes of diaphyseal components, labeled 52A, 52B, 52C, 52D, 52E. The illustrated diaphyseal components 52A, 52B, 52C, 52D, 52E include several common features. In the following description and in the drawings, like parts are identified with the same reference number, followed by a letter designation to identify the particular size of component.

Each of the illustrated diaphyseal components 52A, 52B, 52C, 52D, 52E has an annular first end 54A, 54B, 54C, 54D, 54E an annular second end 56A, 56B, 56C, 56D, 56E and a longitudinal axis 58A, 58B, 58C, 58D, 58E extending from the first annular end 54A, 54B, 54C, 54D, 54E to the second annular end 56A, 56B, 56C, 56D, 56E. Each of the illustrated diaphyseal components 52A, 52B, 52C, 52D, 52E also has an outer surface 60A, 60B, 60C, 60D, 60E.

The outer surface 60A, 60B, 60C, 60D, 60E of each of the illustrated diaphyseal components 52A, 52B, 52C, 52D, 52E, 52F has a first tapered portion 62A, 62B, 62C, 62D, 62E at the first annular end 54A, 54B, 54C, 54D, 54E and a second tapered portion 64A, 64B, 64C, 64D, 64E at the second annular end 56A, 56B, 56C, 56D, 56E.

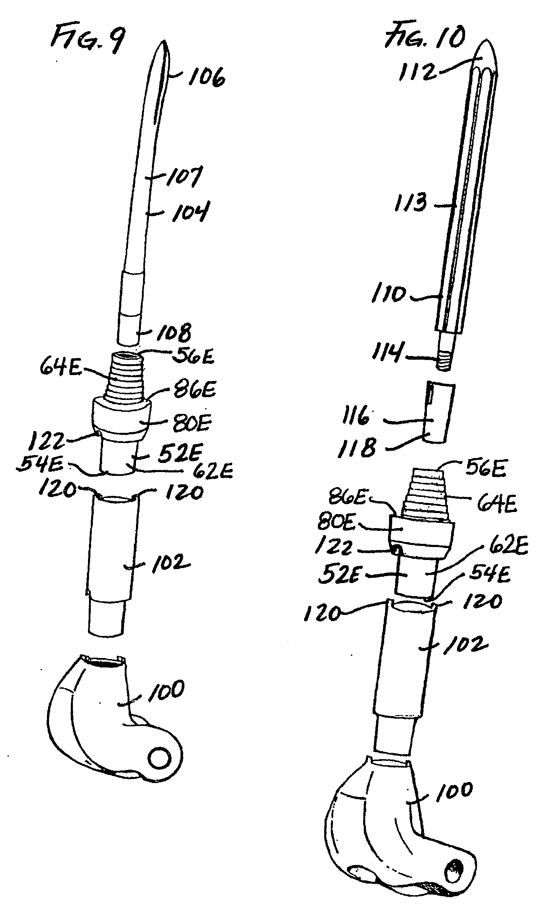

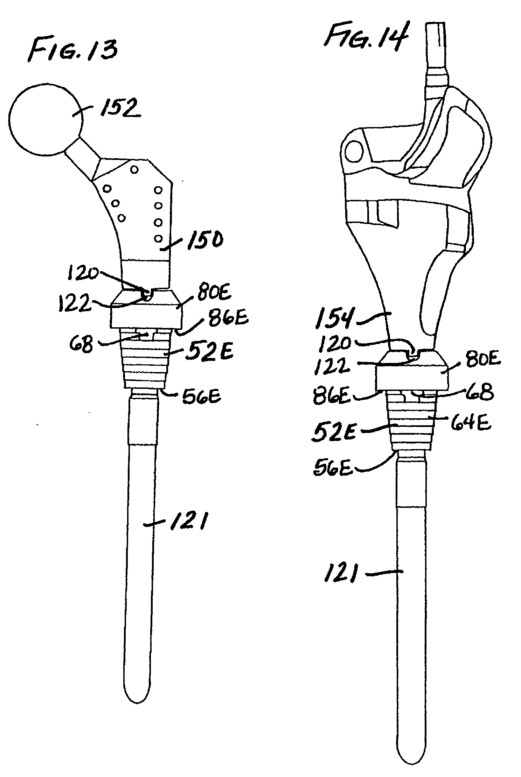

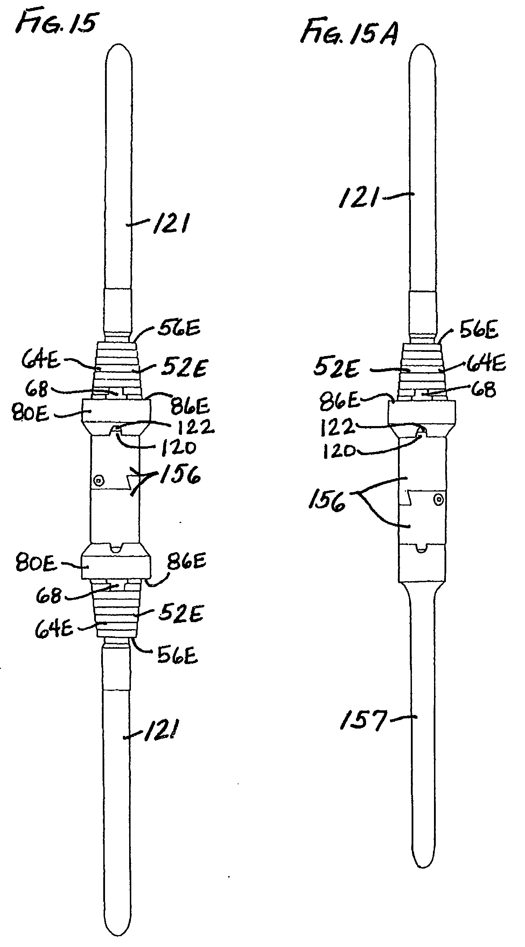

The first tapered portion 62A, 62B, 62C, 62D, 62E of each diaphyseal component 52A, 52B, 52C, 52D, 52E has a minimum outer diameter at the first annular end 54A, 54B, 54C, 54D, 54E and a maximum outer diameter spaced from the first annular end 54A, 54B, 54C, 54D, 54E. The first tapered portion 62A, 62B, 62C, 62D, 62E is smooth, and defines a male Morse taper post, similar to the Morse taper posts described and shown in Modular Limb Preservation System, U.S. Pat. Publication No. US2003/0204267A1. The first tapered portion is sized and shaped to be received within and to lock through a friction fit with a Morse taper bore provided in one of the other implant components of the system, such as a modular articulation component like the distal femoral component shown in FIG. 9-12, the proximal femoral component shown in FIG. 13 or the tibial component shown in FIG. 14. The first tapered portion can also be received within and to frictionally lock with a segmental component such as those shown in FIGS. 9-13 including an intercalary component as shown in FIG. 15. Thus, the diaphyseal components 52A, 52B, 52C, 52D, 52E are each capable of interlocking with other components of the system. Accordingly, the first tapered portion of each of the diaphyseal components is of like shape and size so that any of the sizes of the diaphyseal components can be used with any of the other modular components of the system having the corresponding female taper.

The second tapered portion 64A, 64B, 64C, 64D, 64E of each diaphyseal implant component 52A, 52B, 52C, 52D, 52E in the set 50 is of a different size to accommodate the needs of the individual patient's anatomy. The illustrated set includes sizes ranging from extra-small 52A to extra-large 52E.

The second tapered portion 64A, 64B, 64C, 64D, 64E of each diaphyseal implant component 52A, 52B, 52C, 52D, 52E in the set 50 has a minimum outer diameter at the second end 56A, 56B, 56C, 56D, 56E and a maximum outer diameter spaced from the first annular end 54A, 54B, 54C, 54D, 54E and the second annular end 56A, 56B, 56C, 56D, 56E. The maximum outer diameter is indicated at 66A, 66B, 66C, 66D, 66E in FIGS. 3-4.

The second tapered portion 64A, 64B, 64C, 64D, 64E has a plurality of flats 68A, 68B, 68C, 68D, 68E at the maximum outer diameter 66A, 66B, 66C, 66D, 66E. The flats are provided to help to limit rotation of the diaphyseal components 52A, 52B, 52C, 52D, 52E with respect to the bone after implantation, as described in more detail below. It should be understood that the diaphyseal implant components could be provided without such flats if desired.

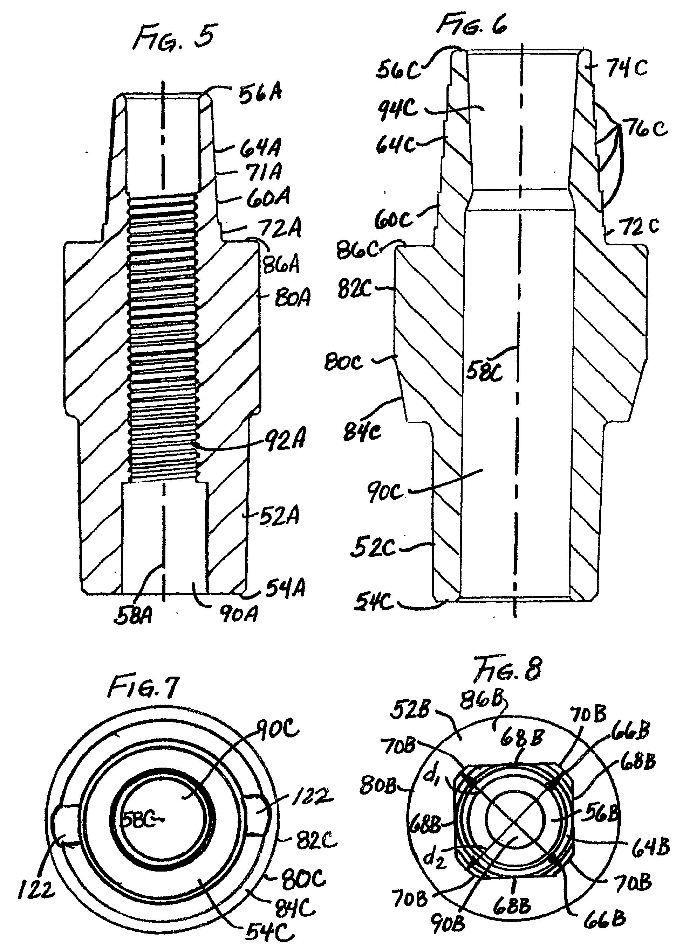

FIG. 8 illustrates an end view of one of the diaphyseal implant components 52B of the set 50, taken from the second end 56B of the component. As there shown, the second tapered portion 64B has four equally spaced flats 68B connected by curved arcs 70B. The maximum outer dimensions of the second tapered portion 64B are shown at d₁ and d₂ in FIG. 8; in the illustrated embodiments, d₁ = d₂. Thus, the second tapered portion 64B has the same outer dimension d₁, d₂ along two perpendicular transverse axes at the maximum outer dimension 66B of the second tapered portion 64B.

In the smallest size of diaphyseal implant component 52A, the outer surface of most of the second tapered portion 64A has a frustoconical shape, shown at 71A in FIGS. 3-4. Frustoconical is intended to mean shaped like the frustum of a cone, that is, it has the shape of the basal part of a solid cone formed by cutting off the top by a plane parallel to the base. In each of the other sizes of diaphyseal implant components 52B, 52C, 52D, 52E in the set 50, the outer surface of the second tapered portion 64B, 64C, 64D, 64E comprises a plurality of annular steps: there is a first annular step 72B, 72C, 72D, 72E between the first end 54B, 54C, 54D, 54E and second end 56B, 56C, 56D, 56E of the diaphyseal implant components, a last annular step 74B, 74C, 74D, 74E at the second end 56B, 56C, 56D, 56E of the diaphyseal implant component and a plurality of intermediate annular steps 76B, 76C, 76D, 76E between the first step 72B, 72C, 72D, 72E and last step 74B, 74C, 74D, 74E. The smallest size of diaphyseal implant component 52A also has a first annular step 72A at its maximum outer diameter 66A adjacent to the frustoconical portion of the second tapered portion 64A.

Each annular step 72A, 72B, 72C, 72D, 72E, 74B, 74C, 74D, 74E, 76B, 76C, 76D, 76E has a substantially cylindrically shaped outer surface and a longitudinal height; the largest diameter steps deviate from a cylindrical shape in the illustrated embodiments because of the presence of the four flats 68. In each illustrated size of diaphyseal implant component, the first annular step 72A, 72B, 72C, 72D, 72E has the greatest maximum outer diameter, and the maximum outer diameter of each step progressively decreases to the last annular step 74B, 74C, 74D, 74E which has the smallest maximum outer diameter. In the illustrated set of diaphyseal implant components 52A, 52B, 52C, 52D, 52E examples of sizes and numbers of steps are provided in the following table:

| Extra Extra Small Diaphyseal Implant Component 52A | | | |
|---|---|---|---|
| | Height | Outer Diameter | Taper Angle |
| First step 72A | 2mm | 12.95mm | - |
| Frustoconical Portion 71A | 15.04mm | 12.65mm maximum to 10.67mm minimum | 3° |

| Extra Small Diaphyseal Implant Component 52B | | | |
|---|---|---|---|
| | Step Height | Step Outer Diameter | Overall Taper Angle |
| First step 72B | 2mm | 15.23mm | 4°52' |
| Second step | 4mm | 14.37mm | |
| Third step | 4mm | 13.51mm | |
| Fourth step | 4mm | 12.65mm | |
| Last step 74B | 4mm | 11.79 mm | |

| Small Diaphyseal Implant Component 52C | | | |
|---|---|---|---|
| | Step Height | Step Outer Diameter | Overall Taper Angle |
| First step 72C | 2mm | 19.09mm | 4°33' |
| Second step | 4mm | 18.37mm | |
| Third step | 4mm | 17.65mm | |
| Fourth step | 4mm | 16.93mm | |
| Fifth step | 4mm | 16.21mm | |
| Last step 74C | 4mm | 15.49mm | |

| Medium Diaphyseal Implant Component 52D | | | |
|---|---|---|---|
| | Step Height | Step Outer Diameter | Overall Taper Angle |
| First step 72D | 2mm | 22.53mm | 6°35' |
| Second step | 4mm | 21.51mm | |
| Third step | 4mm | 20.49mm | |
| Fourth step | 4mm | 19.47mm | |
| Fifth step | 4mm | 18.45mm | |
| Sixth step | 4mm | 17.43mm | |
| Last step 74D | 4mm | 16.41mm | |

| Large Diaphyseal Implant Component 52E | | | |
|---|---|---|---|
| | Step Height | Step Outer Diameter | Overall Taper Angle |
| First step 72E | 2mm | 26.51mm | 6°39' |
| Second step | 4mm | 25.49mm | |
| Third step | 4mm | 24.47mm | |
| Fourth step | 4mm | 23.45mm | |
| Fifth step | 4mm | 22.44mm | |
| Sixth step | 4mm | 21.42mm | |
| Seventh step | 4mm | 20.40mm | |
| Last step 74E | 4mm | 19.38mm | |

In the above table, the Overall Taper Angle refers to the angle defined by a line tangent to the steps 72, 74, 76 and a line parallel to the longitudinal axis 58 in each size.

It should be understood that the sizes, numbers of steps and overall taper angles identified in the above tables are provided as examples only. The present invention is not limited to a stepped configuration or to any particular size, number of steps or overall angle of taper unless expressly called for in the claims. Moreover, although five sizes are illustrated in the set 50, fewer or more sizes could be provided; the invention is not limited to any number of sizes of implant components unless expressly called for in the claims.

In each of the illustrated diaphyseal implant components 52A, 52B, 52C, 52D, 52E, most of the entire second tapered portion, including the entire frustoconical portion of the small implant component 52A to all of the steps of the other sizes of implant components 52B, 52C, 52D, 52E, 52F is porous. The last or smallest diameter step 74 is not porous in these embodiments, and the last 2 mm of the smallest diaphyseal implant 52A is not porous. As used herein, "porous" refers to a surface that is conducive to bone ingrowth for non-cemented fixation, and "smooth" refers to a surface that is not conducive to such bone ingrowth. Suitable porous surfaces can be made by many different methods: casting, embossing, etching, milling, machining, and coating such as by plasma-spraying or by bonding, for example. Bonded materials can comprise sintered metal beads, sintered metal mesh or screen, or sintered metal fibers, for example. Known, commercially available materials and techniques can be used to create the porous tapered surfaces of the diaphyseal components: for example, POROCOAT® coating, available from DePuy Orthopaedics, Inc. of Warsaw, Indiana, could be used, as well as other commercially available coatings. In addition, the porous surfaces may include other materials conducive to bone ingrowth, such as hydroxy apatite coatings, for example. It should be understood that the above-identified examples of materials, methods and commercial products are provided as examples only; the present invention is not limited to any particular material, method or commercial product for the porous surfaces unless expressly called for in the claims. In addition, it should be understood that as additional materials and methods become available to create surfaces that promote bony ingrowth, it is believed that such other materials and methods may also be useful with the present invention.

Each of the four flats 68A, 68B, 68C, 68D, and 68E in each illustrated size is 6mm high. The flats are disposed at 90° intervals around the first step and second step in the diaphyseal implant components 52B, 52C, 52D, 52E, 52F that have stepped second tapered surfaces, and are also disposed at 90° intervals around the tapered frustoconical surface of the smallest implant component. It should be understood that the flats may have different dimensions and different positions.

As shown in FIGS. 3-4, each size of diaphyseal implant components 52A, 52B, 52C, 52D, 52E in the set 50 has an annular collar 80A, 80B, 80C, 80D, 80E disposed between the first tapered portion 62A, 62B, 62C, 62D, 62E and the second tapered portion 64A, 64B, 64C, 64D, 64E of the outer surface 60A, 60B, 60C, 60D, 60E of the implant component. The annular collars 80A, 80B, 80C, 80D, 80E have outer diameters greater than the maximum outer diameter of the first tapered portion 62A, 62B, 62C, 62D, 62E and greater than the maximum outer diameter of the second tapered portion 64A, 64B, 64C, 64D, 64E. In each of the illustrated sizes, at least a portion of the outer surface of each collar is cylindrical in shape: in the extra extra small component 52A, all or substantially all of the outer surface of the collar 80A is cylindrical in shape; in the other sizes 52B, 52C, 52D, 52E the collars 80B, 80C, 8D, 80E include a cylindrical portion 82B, 82C, 82D, 82E adjacent to the second tapered portion 64B, 64C, 64D, 64E and a frustoconical portion 84B, 84C, 84D, 84E adjacent to the first tapered portion 62B, 62C, 62D, 62E. A portion or all of each collar 80A, 80B, 80C, 80D, 80E may be porous; for example, an annular porous strip having a height (longitudinal dimension) of 10 mm may be provided on the cylindrical portions 82A, 82B, 82C, 82D, 82E for soft tissue attachment and ingrowth. Variations in the type and characteristics of the porous coating may be made to encourage soft tissue ingrowth, as opposed to bone ingrowth. Moreover, features may be included on the collar to allow for attachment of soft tissue or the periosteum; for example, suture holes may be provided on the collar.

Each collar 80A, 80B, 80C, 80D, 80E includes a transverse annular surface 86A, 86B, 86C, 86D, 86E that is perpendicular to the longitudinal axis 58A, 58B, 58C, 58D, 58E of the diaphyseal implant component. The transverse annular surface 86A, 86B, 86C, 86D, 86E is sized and provides a surface area sufficient to bear against the resected end of the bone if the diaphyseal implant component subsides. For example, the transverse annular surface 86A, 86B, 86C, 86D, 86E may have an outer diameter in the range of about 1 inch to 1-1/2 inch (25.4 mm to 38.1 mm) and an inner diameter at the first step 72A, 72B, 72C, 72D, 72E in the range of about ½ inch to 1 inch (12.7 mm to 25.4 mm), thus providing surface areas in the range of about 0.59 square inches to about 0.98 square inches (about 380 mm² to about 633 mm²). With a porous coating, the diameters should increase by about sixty-thousandths of an inch (1.5 mm) It should be understood that these dimensions are provided as examples only; the present invention is not limited to any particular dimension unless expressly called for in the claims. The transverse annular surface 86A, 86B, 86C, 86D, 86E may be porous or smooth; if porous, the transverse annular surface may provide a surface conducive to tissue ingrowth:

Representative cross-sections of the diaphyseal implant components 52A, 52B, 52C, 52D, 52E are illustrated in FIGS. 5-6. It should be understood that cross-sections of other sizes of diaphyseal implant components may be similar to those shown in FIGS. 5-6.

Each of the diaphyseal implant components 52A, 52B, 52C, 52D, 52E has a throughbore extending longitudinally through the entire length of the component, from the first annular end 54A, 54B, 54C, 54D, 54E to the second annular end 56A, 56B, 56C, 56D, 56E. Examples of throughbores are shown at 90A, 90B and 90C in FIGS. 5-8. In the extra-extra small and extra-small sizes 52A, 52B, the throughbore has a threaded portion (shown at 92A in FIG. 5) to receive the threaded end of a stem extension. In the other larger sizes 52C, 52D, 52E, the portion of the throughbore near the second annular end 56C, 56D, 56E comprises a Morse taper bore sized and shaped to receive a Morse taper post at the end of a stem extension or at the end of an adapter. An example of such a Morse taper bore is shown at 94C in FIG. 6. It should be understood that the illustrated longitudinal throughbores are provided as examples only; other designs could be employed, depending on the desired wall thickness for the implant and the type of connection to be employed to the other implant components.

FIGS. 9-10 illustrate the large size diaphyseal implant component 52E in exploded views with other modular implant components that may be included in a kit or system and assembled with the diaphyseal implant component 52 for implantation. In FIGS. 9-10, the assembly is intended for use in replacing a portion of the distal femur. The assemblies of both FIGS. 9 and 10 include a distal femoral implant 100, a segmental implant component 102, a diaphyseal implant component 52 (the large size 52E is illustrated).

Each assembly also includes a stem extension. In FIG. 9, the stem extension 104 has a coronal-slotted free end or tip 106, a body 107 and a connection end 108. The connection end 108 comprises a Morse taper post in the embodiment of FIG. 9. The Morse taper post at the connection end 108 is received within and frictionally locks with the Morse taper bore of the diaphyseal implant component 52E. In FIG. 10, the stem extension 110 has a free end or tip 112, a body 113 and a connection end 114 that comprises a male threaded member. The embodiment of FIG. 10 also includes an adapter 116 with a threaded opening (not shown) to receive the male threaded connection end 114 of the stem extension and a Morse taper post 118 to be received in the Morse taper bore of the diaphyseal implant component 52E. All of the large size diaphyseal implant components 52C, 52D, 52E can be assembled with stem extensions in the manners illustrated in FIGS. 9-10. Due to constraints on the thicknesses of the walls of the second tapered portions 64A, 64B of the smaller sized diaphyseal implant components 52A, 52B, accommodation is only made for connection to a stem extension with a threaded male end of the type shown in FIG. 10.

The bodies 107, 113 of the stem extensions 104, 110 may vary. For example, a substantial part of the length of the body, such as body 107 of FIG. 9, can be porous. Alternatively, the body can be sized and shaped for cemented application, like the body 113 of the stem extension 110 of FIG. 10. Alternatively, the body of the stem extension can be splined.

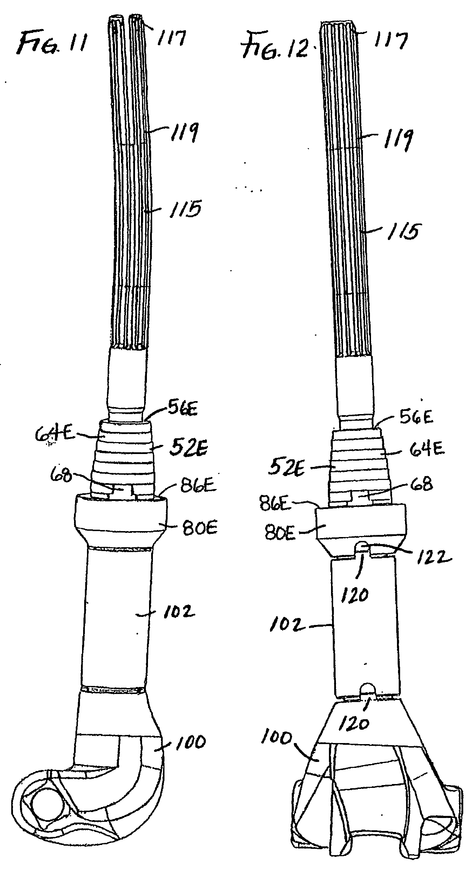

FIGS. 11-12 illustrate a stem extension 115 with a coronal slotted free end 117, a splined body 119, and a connection end (not shown) comprising a Morse taper post. In the embodiment of FIGS. 11-12, the splined body 119 of the stem extension 115 comprises a plurality of cutting flutes. The stem extension 115 of FIGS. 11-12 is not porous. Although in FIGS. 11-12 the free end 117 of the stem extension 115 is illustrated as being substantially flat, it may be desirable for the free end 117 to be bullet-shaped.

Features of the adapter 116 are disclosed in more detail in U.S. Pat. App. Ser. No. 10/817,051 entitled "Modular Implant System with Fully Porous Coated Sleeve", filed on April 2, 2004 by Goodfried, Hazebrouck, Lester and Brown (U.S. Pat. Publication No. US2005/0107883A1).

As disclosed in U.S. Pat. Publication No.US2003/0204267A1, the distal femoral implant component 100 and segmental component 102 both include tabs 120. Each of the diaphyseal implant components 52A, 52b, 52C, 52D, 52E include corresponding notches 122 to receive the tabs 120 to prevent the diaphyseal implant components from rotating. These notches can also be used to separate the components if necessary; a tool such as that disclosed in U.S. Pat. No. 6,786,931 may be used.

It should be understood that a typical implant kit or system would include several sizes of distal femoral implant components 100, segmental components 102 and stem extensions 104, 110. It should also be understood that depending on the size and shape of the distal femoral component, it may not be necessary to use a segmental component 102; the diaphyseal implant component 52A, 52B, 52C, 52D, 52E could be connected directly to the femoral implant component 100.

Use of the diaphyseal implant components 52A, 52B, 52C, 52D, 52E of the present invention is not limited to segmental components and femoral components. As illustrated in FIGS. 13-15A, the diaphyseal implant components of the present invention can be used with other implant components having an articulation portion. For example, as shown in FIG. 13, the articulation portion of the implant component could comprise a proximal femoral component 150 (including a femoral head 152). As shown in FIG. 14 the articulation portion of the implant component could comprise a proximal tibia component 154 or other component, such as a proximal humeral component (not shown).

As shown in FIGS. 15-15A, the implant component could be an intercalary implant instead of an articulation component. FIG. 15 illustrates two large size diaphyseal implant components 52E in use with a two-piece intercalary implant 156 of the type disclosed in U.S. App. Ser. No. 10/403,612 entitled "Intercalary Prosthesis, Kit and Method," filed March 31, 2003 by Hazebrouck (U.S. Pat. Publication No. US2004/0193268A1), or those disclosed in U.S. App. Ser. No. Serial No. 10/403,357 entitled "Intercalary Implant," filed on March 31, 2003 by Natalie Heck and Michael C. Jones (U.S. Pat. Publication No. US2004/0193267A1). Such implants may be used with intercalary trials such as those disclosed in U.S. Pat. App. Ser. No. 10/952,581, entitled "Orthopaedic Spacer," filed on September 24, 2004 by Hazebrouck (U.S. Pat. Publication No. US2005/0107794A1). FIG. 15A illustrates a single diaphyseal implant components in use with the two-piece intercalary component 156 and a standard stem extension 157 for the LPS implant system.

In FIGS. 13-15A the stem extension is shown diagrammatically and indicated generally by reference number 121, with the free end indicated by reference number 123. Other than the bullet shape of the free end 123, no other features are shown for the body 125 of the stem extension. It should be understood that the body 125 of the stem extension 121 in any of FIGS. 13-15A could have any of the above described features, such as splined cutting flutes, a porous coating, a coronally slotted free end, or could be designed for cemented application.

All of the components of the illustrated implant systems can be made of standard materials for such implants, such as titanium and cobalt-chrome alloys.

It should be understood that although the principles of the present invention are described and illustrated with reference to implant components available from DePuy Orthopaedics, Inc., the invention is not limited to these components or their features. The principles of the present invention can be applied to other implant components, including those of other manufacturers and those subsequently developed.

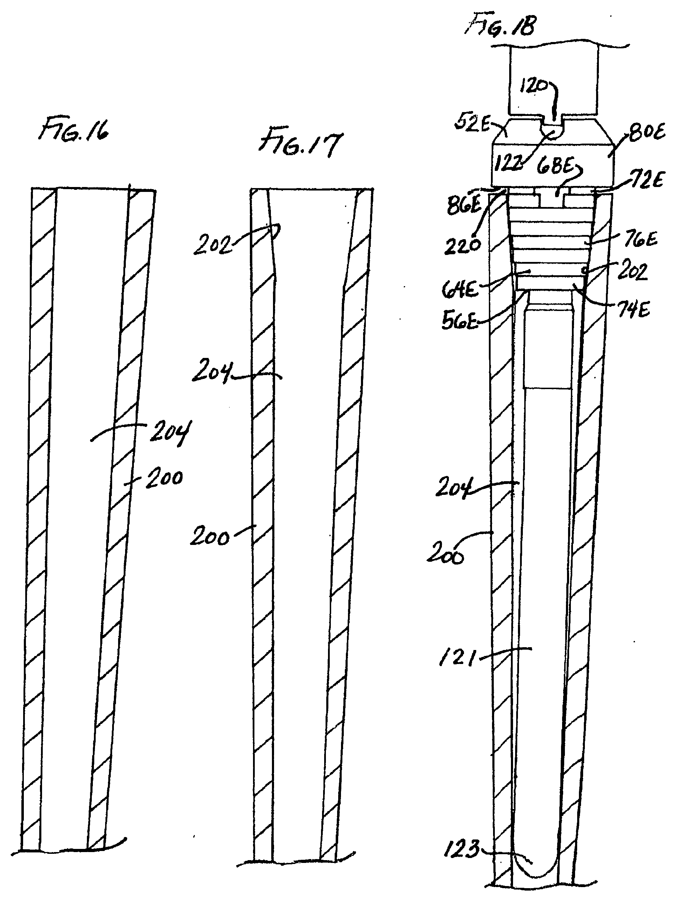

In use, depending on the condition of the native bone tissue, the orthopaedic surgeon will determine the amount of bone to be resected from the femur (or other long bone). Commercially available instrumentation can be used to resect the bone in the appropriate manner. The diaphysis of a resected bone is illustrated in FIGS. 16-18 at 200. If it is desirable to use a diaphyseal implant component 52A, 52B, 52C, 52D, 52E to secure the implant in place, the surgeon can then select an appropriate size of diaphyseal implant component 52A, 52B, 52C, 52D or 52E for the individual patient. The diaphysis 200 of the bone can then be prepared to receive the selected diaphyseal implant component 52A, 52B, 52C, 52D or 52E. The surgeon can use a conical reamer (not shown) of a size and shape matching the size and shape of the selected diaphyseal component to mill or machine the diaphysis 200 of the bone to create a tapered bore that closely matches the size and shape of the second tapered surface 64A, 64B, 64C, 64D, 64E of the selected diaphyseal implant component. A tapered bore is illustrated in FIGS. 17-18 at 202. Since the tapered bore is created to match the size and shape of the selected diaphyseal implant component, the implants and techniques of the present invention are adaptable to different patient anatomies.

The stem extension and part of the diaphyseal implant component of the assembled implant, can then be inserted into the bone as illustrated in FIG. 18 and positioned with the tip or free end of the stem extension engaging the bone surface of the intramedullary canal 204 and with the second tapered portion 64A, 64B, 64C, 64D or 64E bearing against the tapered diaphyseal bone defining the tapered bore 202. The stem extension in FIG. 18 is identified with reference number 121 and its free end is identified with reference number 123; as discussed above with respect to FIGS. 13-15A, the stem extension 121 is illustrated diagrammatically, and can include any of the features of the stem extensions 104, 110, 115 described above. Because of the shapes and textures of the implant components 121, 52A, 52B, 52C, 52D or 52E, there should be no binding before the diaphyseal component 52A, 52B, 52C, 52D or 52E is fully seated. Accordingly, implantation should be relatively easy.

Generally, when implanted, the first step 72A, 72B, 72C, 72D, 72E of each of the diaphyseal implant components 52A, 52B, 52C, 52D, 52E will be exposed outside of the bone as shown in FIG. 18. Subsequently, some subsidence of the implant can occur over time without damage to the bone. The flats 68E prevent the diaphyseal component 52E from rotating or turning in the tapered bore 202 that the surgeon created for it.

As shown in FIG. 18, when fully seated, the implant assembly contacts the bone at both the tip 123 of the stem extension 121 and at the second tapered outer surface 64E of the diaphyseal component 52E. Bone ingrowth can occur around the entire second tapered portion 64E of the diaphyseal implant component 52E. Depending on the intramedullary canal anatomy and characteristics of the stem extension, bone ingrowth can also occur along all or part of the body of the stem; for example, bone ingrowth could occur at the free end of the stem extension and/or at any area between the diaphyseal component and the free end of the stem. For example, if a cemented stem extension is used, such as the stem extension 110 of FIG. 10, there should be no bone ingrowth along the body of the stem. Similarly, no substantial bone ingrowth should occur along the stem with the splined stem extension 115 of FIGS. 11-12. If all or part of the stem extension 104 of FIG. 9 is porous, bone ingrowth can be expected at the porous area.

With the stepped designs of the larger diaphyseal implant components, such as diaphyseal implant components 52B, 52C, 52D, 52E, shear forces are essentially converted to compressive loads, and the compressive loads are spread among the steps 74, 76 contacting the diaphyseal bone defining the tapered bore 202. Accordingly, the implant is immediately stable and capable of bearing weight. In addition, with the bone bearing the axial load at the diaphyseal bone defining the tapered bore 202, there is no disadvantageous stress shielding of the bone. Moreover, with the implant assembly contacting the bone at both the tip 106 of the stem extension and at the contacting surfaces of the tapered bore 202 and second tapered surface 64, any moment arm is significantly reduced if not eliminated. With bone ingrowth occurring at both spaced locations over time, long term implant stability should be improved. Accordingly, the implant assembly of the present invention should be less likely to loosen over time.

As can be seen in FIG. 18, a small gap 220 may be between the exposed resected bone surface and the transverse annular surface 86E of the collar 80E portion of the component 52E when implanted. If the implant does subside, this gap can decrease to the point that the transverse annular surface 86E bears directly against the exposed resected bone surface. If the transverse annular surface is porous, tissue ingrowth can occur in the gap 220 over time to seal the intramedullary canal 204 against debris.

With any of the illustrated diaphyseal implant components, the periosteum of the bone should grow into the porous outer surface of the collar portion 80 of the diaphyseal implant component 52. Essentially the ingrowth of tissue along the cylindrical outer surface of the collar (or along the exposed portion of the transverse annular surface of the collar) should effectively seal off the intramedullary canal 204, to thereby protect the patient from injury or disease resulting from debris entering into the intramedullary canal.

Moreover, with the modular implant system of the present invention, it should be possible to reduce inventory of the necessary parts in an implant system or kit.

It should also be understood that a typical surgical kit would also include trial implant components (not shown) like those shown in FIGS. 3-4 and 9-15. The surgeon would typically assemble a trial implant and temporarily secure the trial implant assembly in place on the prepared diaphyseal bone to ensure that the assembled implant will be the optimum for the individual patient's needs. The trial components can have features like those described above for the final implant components.

In case it is necessary to ultimately remove the implant assembly from the patient, such removal should not require the removal of excessive bone stock, since it should only be necessary to remove the portion of the diaphysis defining the tapered bore 202.

Various modifications and additions can be made to the above-described embodiments without departing from the scope of the invention provided that all such modifications and additions are intended to fall within the scope of the invention as defined by the appended claims.

## Claims

1. A diaphyseal implant component comprising:
a. a first end (54A, 54B, 54C, 54D, 54E),
b. a second end (56A, 56B, 56C, 56D, 56E) with a bore (90A, 90B, 90C, 90D, 90E) which is tapered,
c. a longitudinal axis (58A, 58B, 58C, 58D, 58E) extending from the first end to the second end, and
d. an outer surface (60A, 60B, 60C, 60D, 60E) including a first tapered portion (62A, 62B, 62C, 62D, 62E) at the first end, the first tapered portion of the outer surface being smooth and having a minimum outer dimension at the first end and a maximum outer dimension spaced from the first end, and
e. an annular collar (80A, 80B, 80C, 80D, 80E) having an outer diameter greater than the maximum outer dimension of the first tapered portion of the outer surface,
**characterised in that** the outer surface of the component includes a second tapered portion (64A, 64B, 64C, 64D, 64E) at the second end which is porous and has a minimum outer dimension at the second end and a maximum outer dimension spaced from the first end and second end, and the same outer dimension along two perpendicular transverse axes at the maximum outer dimension of the second tapered portion,
the tapered bore in the second end of the component has a maximum inner dimension at the second end and a minimum inner dimension spaced between the first end and the second end of the implant component, and
the annular collar is disposed between the first tapered portion of the outer surface and the second tapered portion of the outer surface, the annular collar having an outer diameter which is greater than the maximum outer dimension of the second tapered portion of the outer surface.

2. The diaphyseal implant component of claim 1, in which at least a portion of the outer surface of the collar (80A, 80B, 80C, 80D, 80E) is porous.

3. The diaphyseal implant component of claim 1, in which the collar (80A, 80B, 80C, 80D, 80E) has an outer surface including a portion having a cylindrical shape.

4. The diaphyseal implant component of claim 3, in which the outer surface of the collar (80A, 80B, 80C, 80D, 80E) includes a portion having a frustoconical shape.

5. The diaphyseal implant component of claim 4, in which at least a portion of the cylindrically-shaped outer surface of the collar (80A, 80B, 80C, 80D, 80E) is porous.

6. The diaphyseal implant component of claim 1, in which the second tapered portion (64A, 64B, 64C, 64D, 64E) of the outer surface of the implant component includes a first step between the first end of the implant component and the second end of the implant component, a last step at the second end of the implant component and an intermediate step between the first step and last step, each step having a maximum outer dimension and a longitudinal height, the maximum outer dimension of the second tapered portion of the implant component being at the first step and the minimum outer dimension of the second tapered portion of the implant component being at the last step.

7. The diaphyseal implant component of claim 6, in which the height of the first step is less than the height of the last step and less than the height of the intermediate step.

8. The diaphyseal implant component of claim 7, in which the height of the first step is about 2 mm.

9. The diaphyseal implant component of claim 6, in which the surface of each step is fully porous coated.

10. The diaphyseal implant component of claim 1, in which the implant component is part of a kit including a plurality of diaphyseal implant components of different size.

11. The diaphyseal implant component of claim 1, in which the diaphyseal implant component is part of a kit including a stem for attachment to the second end of the diaphyseal implant component and another implant component having a different shape than the stem and the diaphyseal implant component for attachment to the first end of the diaphyseal implant component.

## Patentansprüche

1. Diaphysäre Implantatkomponente, die umfasst:
a. eine erstes Ende (54A, 54B, 54C, 54D, 54E),
b. ein zweites Ende (56A, 56B, 56C, 56D, 56E) mit einer Bohrung (90A, 90B, 90C, 90D, 90E), die angeschrägt ist,
c. eine Längsachse (58A, 58B, 58C, 58D, 58E), die sich von dem ersten Ende zu dem zweiten Ende erstreckt, und
d. eine Außenfläche (60A, 60B, 60C, 60D, 60E), die einen ersten angeschrägten Abschnitt (62A, 62B, 63D, 62D, 62E) an dem ersten Ende umfasst, wobei der erste angeschrägte Abschnitt der Außenfläche glatt ist und an dem ersten Ende eine minimale Außenabmessung und beabstandet von dem ersten Ende eine maximale Außenabmessung hat, und
e. eine ringförmige Manschette (80A, 80B, 80C, 80D, 80E) mit einem Außendurchmesser, der größer als die maximale Außenabmessung des ersten angeschrägten Abschnitts der Außenfläche ist,
**dadurch gekennzeichnet, dass** die Außenfläche der Komponente einen zweiten angeschrägten Abschnitt (64A, 64B, 64C, 64D, 64E) an dem zweiten Ende umfasst, der porös ist und an dem zweiten Ende eine minimale Außenabmessung und von dem ersten Ende und dem zweiten Ende beabstandet eine maximale Außenabmessung und entlang zwei Querachsen an der maximalen Außenabmessung des zweiten angeschrägten Abschnitts die gleiche Außenabmessung hat,
die angeschrägte Bohrung in dem zweiten Ende der Komponente an dem zweiten Ende eine maximale Innenabmessung und beabstandet zwischen dem ersten Ende und dem zweiten Ende der Implantatkomponente eine minimale Innenabmessung hat, und
die ringförmige Manschette zwischen dem ersten angeschrägten Abschnitt der Außenfläche und dem zweiten angeschrägten Abschnitt der Außenfläche angeordnet ist, wobei die ringförmige Manschette einen Außendurchmesser hat, der größer als die maximale Außenabmessung des zweiten angeschrägten Abschnitts der Außenfläche ist.

2. Diaphysäre Implantatkomponente nach Anspruch 1, wobei wenigstens ein Abschnitt der Außenfläche der Manschette (80A, 80B, 80C, 80D, 80E) porös ist.

3. Diaphysäre Implantatkomponente nach Anspruch 1, wobei die Manschette (80A, 80B, 80C, 80D, 80E) eine Außenfläche hat, die einen Abschnitt mit einer zylindrischen Form umfasst.

4. Diaphysäre Implantatkomponente nach Anspruch 3, wobei die Außenfläche der Manschette (80A, 80B, 80C, 80D, 80E) einen Abschnitt mit einer Kegelstumpfform umfasst.

5. Diaphysäre Implantatkomponente nach Anspruch 4, wobei wenigstens ein Abschnitt der zylindrisch geformten Außenfläche der Manschette (80A, 80B, 80C, 80D, 80E) porös ist.

6. Diaphysäre Implantatkomponente nach Anspruch 1, wobei der zweite angeschrägte Abschnitt (64A, 64B, 64C, 64D, 64E) der Außenfläche der Implantatkomponente eine erste Stufe zwischen dem ersten Ende der Implantatkomponente und dem zweiten Ende der Implantatkomponente, eine letzte Stufe an dem zweiten Ende der Implantatkomponente und eine Zwischenstufe zwischen der ersten Stufe und der letzten Stufe umfasst, wobei jede Stufe eine maximale Außenabmessung und eine Höhe in der Längsrichtung hat, wobei die maximale Außenabmessung des zweiten angeschrägten Abschnitts der Implantatkomponente an der ersten Stufe ist und die minimale Außenabmessung des zweiten angeschrägten Abschnitts der Implantatkomponente an der letzten Stufe ist.

7. Diaphysäre Implantatkomponente nach Anspruch 6, wobei die Höhe der ersten Stufe kleiner als die Höhe der letzten Stufe und kleiner als die Höhe der Zwischenstufe ist.

8. Diaphysäre Implantatkomponente nach Anspruch 7, wobei die Höhe der ersten Stufe etwa 2 mm ist.

9. Diaphysäre Implantatkomponente nach Anspruch 6, wobei die Fläche jeder Stufe vollkommen porös beschichtet ist.

10. Diaphysäre Implantatkomponente nach Anspruch 1, wobei die Implantatkomponente Teil eines Bausatzes ist, der eine Vielzahl von diaphysären Implantatkomponenten verschiedener Größe enthält.

11. Diaphysäre Implantatkomponente nach Anspruch 1, wobei die diaphysäre Implantatkomponente Teil eines Bausatzes ist, der einen Schaft für die Befestigung an dem zweiten Ende der diaphysären Implantatkomponente und eine andere Implantatkomponente mit einer zu dem Schaft und der diaphysären Implantatkomponente unterschiedlichen Form für die Befestigung an dem ersten Ende der diaphysären Implantatkomponente umfasst.

## Revendications

1. Composant d'implant diaphysaire comprenant :
a. une première extrémité (54A, 54B, 54C, 54D, 54E),
b. une seconde extrémité (56A, 56B, 56C, 56D, 56E) ayant un alésage (90A, 90B, 90C, 90D, 90E) qui est conique,
c. un axe longitudinal (58A, 58B, 58C, 58D, 58E) s'étendant de la première extrémité à la seconde extrémité, et
d. une surface externe (60A, 60B, 60C, 60D, 60E) comprenant une première partie conique (62A, 62B, 62C, 62D, 62E) à la première extrémité, la première partie conique de la surface externe étant lisse et ayant une dimension externe minimale à la première extrémité et une dimension externe maximale espacée de la première extrémité, et
e. un collier annulaire (80A, 80B, 80C, 80D, 80E) ayant un diamètre externe supérieur à la dimension externe maximale de la première partie conique de la surface externe,
**caractérisé en ce que** la surface externe du composant comprend une seconde partie conique (64A, 64B, 64C, 64D, 64E) à la seconde extrémité qui est poreuse et a une dimension externe minimale à la seconde extrémité et une dimension externe maximale espacée de la première extrémité et de la seconde extrémité, et la même dimension externe le long de deux axes transversaux perpendiculaires à la dimension externe maximale de la seconde partie conique,
l'alésage conique dans la seconde extrémité du composant a une dimension interne maximale à la seconde extrémité et une dimension interne minimale espacée entre la première extrémité et la seconde extrémité du composant d'implant, et
le collier annulaire est disposé entre la première partie conique de la surface externe et la seconde partie conique de la surface externe, le collier annulaire ayant un diamètre externe qui est supérieur à la dimension externe maximale de la seconde partie conique de la surface externe.

2. Composant d'implant diaphysaire selon la revendication 1, dans lequel au moins une partie de la surface externe du collier (80A, 80B, 80C, 80D, 80E) est poreuse.

3. Composant d'implant diaphysaire selon la revendication 1, dans lequel le collier (80A, 80B, 80C, 80D, 80E) a une surface externe comprenant une partie ayant une forme cylindrique.

4. Composant d'implant diaphysaire selon la revendication 3, dans lequel le collier (80A, 80B, 80C, 80D, 80E) a une surface externe comprenant une partie ayant une forme tronconique.

5. Composant d'implant diaphysaire selon la revendication 4, dans lequel au moins une partie de la surface externe de forme cylindrique du collier (80A, 80B, 80C, 80D, 80E) est poreuse.

6. Composant d'implant diaphysaire selon la revendication 1, dans lequel la seconde partie conique (64A, 64B, 64C, 64D, 64E) de la surface externe du composant d'implant comprend un premier gradin entre la première extrémité du composant d'implant et la seconde extrémité du composant d'implant, un dernier gradin à la seconde extrémité du composant d'implant et un gradin intermédiaire entre le premier gradin et le dernier gradin, chaque gradin ayant une dimension externe maximale et une hauteur longitudinale, la dimension externe maximale de la seconde partie conique du composant d'implant étant au niveau du premier gradin et la dimension externe minimale de la seconde partie conique du composant d'implant étant au niveau du dernier gradin.

7. Composant d'implant diaphysaire selon la revendication 6, dans lequel la hauteur du premier gradin est inférieure à la hauteur du dernier gradin et inférieure à la hauteur du gradin intermédiaire.

8. Composant d'implant diaphysaire selon la revendication 7, dans lequel la hauteur du premier gradin est d'environ 2 mm.

9. Composant d'implant diaphysaire selon la revendication 6, dans lequel la surface de chaque gradin est pourvue d'un revêtement totalement poreux.

10. Composant d'implant diaphysaire selon la revendication 1, dans lequel le composant d'implant fait partie d'un kit comprenant une pluralité de composants d'implant diaphysaire de tailles différentes.

11. Composant d'implant diaphysaire selon la revendication 1, dans lequel le composant d'implant diaphysaire fait partie d'un kit comprenant une tige pour fixation à la seconde extrémité du composant d'implant diaphysaire et un autre composant d'implant ayant une forme différente de celle de la tige et du composant d'implant diaphysaire pour fixation à la première extrémité du composant d'implant diaphysaire.
